(19)

Europäisches
Patentamt
European
Patent Office
Office européen
des brevets

(11) **EP 4 142 605 B1**

(12) **EUROPEAN PATENT SPECIFICATION**

(45) Date of publication and mention
of the grant of the patent:
**11.06.2025 Bulletin 2025/24**

(21) Application number: **21719571.8**

(22) Date of filing: **14.04.2021**

(51) International Patent Classification (IPC):
***A61B 8/06*** *(2006.01)*     ***A61B 8/08*** *(2006.01)*
***A61B 8/14*** *(2006.01)*     ***A61B 8/00*** *(2006.01)*

(52) Cooperative Patent Classification (CPC):
**A61B 8/06; A61B 8/0891; A61B 8/145;
A61B 8/463; A61B 8/488; A61B 8/5223;
G01S 7/52074; G01S 15/8925; G01S 15/8984;
G01S 15/8988; G01S 15/8993;** A61B 8/0883;
A61B 8/4488; A61B 8/467; A61B 8/5246;    (Cont.)

(86) International application number:
**PCT/EP2021/059617**

(87) International publication number:
**WO 2021/219372 (04.11.2021 Gazette 2021/44)**

(54) **THREE DIMENSIONAL COLOR DOPPLER FOR ULTRASONIC VOLUME FLOW MEASUREMENT**

DREIDIMENSIONALER FARBDOPPLER ZUR
ULTRASCHALL-VOLUMENDURCHFLUSSMESSUNG

DOPPLER COULEUR TRIDIMENSIONNEL POUR MESURE DE DÉBIT VOLUMIQUE PAR
ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.04.2020 US 202063015879 P**

(43) Date of publication of application:
**08.03.2023 Bulletin 2023/10**

(73) Proprietor: **Koninklijke Philips N.V.
5656 AG Eindhoven (NL)**

(72) Inventors:
 • **JAGO, James, Robertson
 5656 AE Eindhoven (NL)**
 • **LOUPAS, Thanasis
 5656 AE Eindhoven (NL)**

(74) Representative: **Philips Intellectual Property &
Standards
High Tech Campus 52
5656 AG Eindhoven (NL)**

(56) References cited:
**EP-A2- 0 842 638**     **US-A- 5 701 898**
**US-A- 5 769 079**     **US-A- 6 071 242**
**US-A1- 2002 013 530**     **US-A1- 2014 013 849**
**US-A1- 2015 250 453**     **US-A1- 2020 174 119**
**US-B1- 6 293 914**

 • **DUNMIRE B ET AL: "Cross-beam vector Doppler
 ultrasound for angle-independent velocity
 measurements", ULTRASOUND IN MEDICINE
 AND BIOLOGY, NEW YORK, NY, US, vol. 26, no.
 8, 1 October 2000 (2000-10-01), pages 1213 -
 1235, XP004295674, ISSN: 0301-5629, DOI:
 10.1016/S0301-5629(00)00287-8**

      **(Cont. next page)**

**EP 4 142 605 B1**

(52) Cooperative Patent Classification (CPC): (Cont.)
     G01S 7/52073; G01S 15/8981

**Description**

This invention relates to medical diagnostic ultrasound systems and, in particular, to ultrasound systems which produce quantified measurements of the volume flow of blood through the heart or a blood vessel.

Ultrasound has long been used to assess various parameters of blood flow in the heart and vascular system using the Doppler principle. The basic Doppler response is flow velocity, which can further be used to determine additional characteristics of blood flow. One characteristic of interest to cardiologists is the volume flow of blood through a vessel. Early efforts to estimate volume flow consisted of multiplying a measurement of the mean velocity of blood flow by the nominal cross-sectional area of a blood vessel. However, these early efforts had shortcomings due to the need to make certain estimates. One is that the vessel lumen is circular. Another is the estimation of the mean velocity from a single Doppler measurement or from a qualitative assessment of spectral Doppler data. Velocity measurement must also be corrected for the angle between the ultrasound beam direction and the direction of flow. Yet another consideration is the laminar flow profile in the presence of stenosis.

A further complication arises due to the pulsatility of arterial flow. While venous flow is substantially constant, arterial flow is constantly changing over the heart cycle. Thus, the standard techniques often lack for user independency and repeatability. Some of these demands have been eased by the advent of 3D ultrasound to assess flow conditions and particularly its ability to acquire volume blood flow information. With 3D imaging, the full vessel lumen can be imaged and a sequence of 3D image data sets acquired for later replay and diagnosis. When data of the full volumetric flow in the vessel is acquired in the data sets, the image data can be examined during post-acquisition diagnosis to assess the flow profile. Different 2D image planes can be extracted from the 3D data in multi-planar reconstruction (MPR), so that an image plane of a desired orientation through a vessel can be examined. Three dimensional imaging thus addresses many of the static imaging challenges which are problematic with 2D flow estimation. However, a substantial amount of time can be required to acquire Doppler data in three dimensions, reducing the temporal accuracy of analyzing volume flow. Accordingly, it is desirable to develop more robust techniques for accurately assessing volume flow in the presence of flow pulsatility and erratic heartbeats. It is further desirable to improve the accuracy and reliability of Doppler angle measurements needed to refine the accuracy of Doppler flow velocity values.

US 5769079 A describes a method for correction of Doppler parameters to account for the Doppler angle between the actual flow and an interrogating scan line.

EP 0842638 describes an ultrasonic diagnostic imaging system for making flow measurements, wherein a user places a cursor over the region of an imaged vessel where a flow measurement is to be made. Velocity measurements and vessel diameter are used to compute volume flow of blood.

US 2015/250453 describes an ultrasound system operable in biplane mode to display two color Doppler biplane images of a vessel, wherein the two image planes intersect along a Doppler beam line used for pulsed wave Doppler.

In accordance with the principles of the present disclosure a diagnostic ultrasound system is described which uses a 3D imaging probe to make volume flow measurements. The probe is preferably a two dimensional matrix array probe which is operated in the biplane mode. One of the imaging planes is manipulated so as to acquire a long axis view of a vessel where volume flow is to be measured. The plane of the other biplane image is aligned with the beam direction of the first image, and images the target vessel obliquely in a transverse view. The beam direction of the longitudinal view image and the flow direction seen in the long axis view thus determine the Doppler angle for Doppler angle correction of velocity values obtained from the transverse image. The Doppler data acquisition rate is relatively high, since only two planar images need to be acquired instead of a full 3D volume acquisition. And Doppler angle correction proceeds directly from the Doppler angle found for the longitudinal image. Known methods of volume flow measurement such as the Gaussian surface integral method can thus be used with high accuracy and repeatability.

In a method of the present disclosure an ultrasonic diagnostic imaging system is used to conduct an ultrasound exam to measure volume flow. Scanning is performed with an ultrasound probe adapted to operate in a biplane mode to acquire a first Doppler image of a target vessel in a long axis view. Scanning is performed with the ultrasound probe in the biplane mode to simultaneously acquire a second Doppler image in a transverse view of the target vessel in an image plane aligned with a Doppler angle of the first image. The two images are displayed simultaneously. Angle correction is performed in accordance with a Doppler beam direction and a flow direction of the first Doppler image. Volume flow is calculated from data of the second Doppler image using angle correction determined from the first Doppler image.

The invention is defined in the independent claims 1,10 and 14. Preferred embodiments are defined in the dependent claims.

In the drawings:

FIGURE 1 illustrates the acquisition of Doppler data for volume flow assessment using pulsed Doppler with uniform insonation.

FIGURES 2a and 2b illustrate the acquisition of Doppler data for volume flow assessment using the Gaussian surface integration method.

FIGURE 3 illustrates the projection of Doppler data from a Gaussian surface acquisition onto a two-dimensional image plane for quantification of volume flow.

FIGURE 4 illustrates the acquisition of Doppler data for volume flow assessment by a transverse color Doppler method.

FIGURE 5 illustrates the projection of biplane image planes from a 2D array transducer.

FIGURES 6 illustrates the acquisition of Doppler data from a vessel for volume flow assessment in accordance with the principles of the present invention.

FIGURE 7 illustrates side-by-side biplane ultrasound images during volume flow Doppler data acquisition in accordance with the present invention.

FIGURE 7a illustrates the transverse view image of FIGURE 7 with the vessel lumen segmented by a circle template.

FIGURE 8 illustrates in block diagram form an ultrasonic diagnostic imaging system constructed in accordance with the principles of the present invention.

FIGURE 8a illustrates the detailed operation of the volume flow calculator of FIGURE 8.

FIGURE 9 is a flowchart of a method for measuring volume flow in accordance with the principles of the present invention.

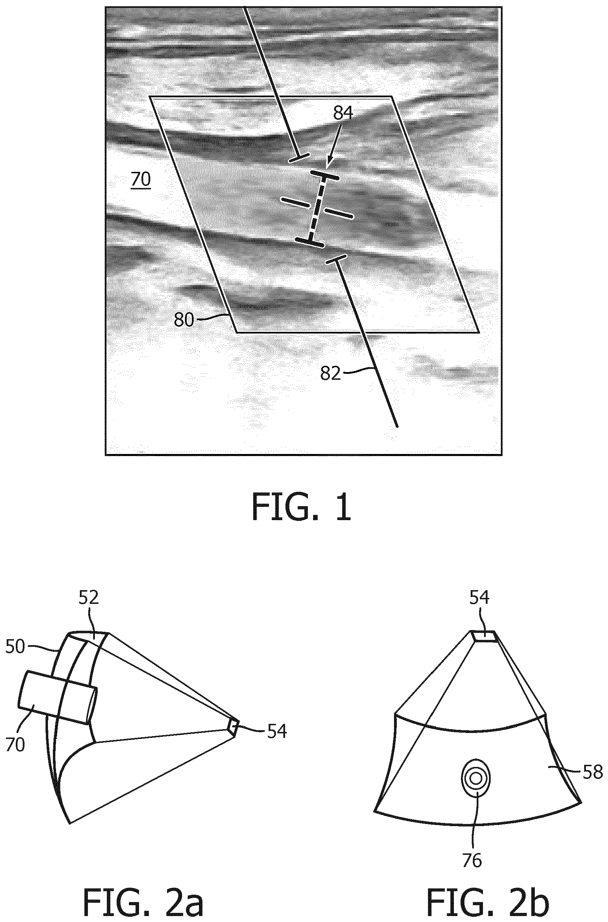

Referring first to FIGURE 1, an ultrasound image is shown depicting the acquisition of Doppler data for volume flow assessment using pulsed Doppler with uniform insonation. This is probably the most widely accepted method in clinical use, because it is available on most commercial ultrasound systems and only requires a one-dimensional (1D) array transducer. The probe operates to alternately acquire B mode echoes for production of a structural image of the tissue and blood vessels, and Doppler echoes for spatial flow velocity depiction inside a color box. The method uses pulsed Doppler ultrasound, whereby a long Doppler sample is placed at an angle to the vessel of interest and volume flow is calculated from the time-average mean velocity of the blood flow. FIGURE 1 illustrates an ultrasound image of the method, which shows a target blood vessel 70 in which volume flow is to be measured. Doppler acquisition is performed in a color box 80 which is seen to be tilted from upper left to the low right, the direction of Doppler beam transmission, and a Doppler gate line 82 is aligned with this beam direction. The color box is Doppler-scanned at the angle shown by parallel scanlines, a technique known as steered linear scanning. The distance of the break in the Doppler gate line 82, with the vessel 70 spanned by the break, establishes the long Doppler sample across the lumen of the vessel. An adjustable flow cursor 84 is positioned over the vessel, with the top and bottom of the cursor positioned at the wall of the vessel and the intermediate horizontal lines aligned with the direction of flow. The angle between the Doppler gate line 82 and the horizontal lines of the flow cursor 84 is the angle used for Doppler angle correction.

However, while still widely used clinically, this method is known to be imprecise and inaccurate due to several incorrect assumptions and measurement dependencies. See, e.g., R. W. Gill, Measurement of blood flow by ultrasound: accuracy and sources of error, Ultrasound in Medicine and Biology, vol. 11 (4), at pp 625-641 (1985). One assumption implicit in the method is that the vessel is uniformly insonated by the ultrasound beam. Since the ultrasound beam is generally smaller than the vessel in elevation, this assumption is typically not valid. If uniform insonation cannot be assumed, then a simplifying assumption must be made that the vessel cross-section is circular. This is typically true only for large arteries and usually is not true for veins. Another implicit assumption is that the temporal sampling rate of the flow is fast enough to capture the variation of flow velocity (and hence volume) through the cardiac cycle. For the pulsed Doppler method, this assumption is usually valid since the temporal sampling rate of a 1D array probe is typically adequate even for very pulsatile flow.

In addition, the accuracy of the measurement is very dependent on accurately determining the Doppler angle and the vessel diameter. The accuracy of the vessel diameter is important, as the diameter is used to determine the vessel cross-sectional area by calculating the vessel cross-sectional area by the equation $Area = \pi r^2$ and then multiplying the area by the flow velocity as corrected by the Doppler angle to estimate volume flow. Accurately determining the Doppler angle is relatively easy for a straight, superficial vessel, but more difficult for bending or deeper vessels. The volume measurement is particularly sensitive to the vessel diameter measurement since the diameter is used to determine the cross-sectional area by means of the above square law.

Other methods for assessing volume flow have been proposed that have less dependency on these assumptions and measurements. One such method is the Gaussian surface integration method, which uses 3D/4D color Doppler and Gauss's law. With this method, the flow volume is determined by integrating (summing) all the color flow voxels over a coronal surface that intersects the target vessel and is perpendicular to the 3D (or 4D) color Doppler beams. See O.D. Kripfgans et al., Measurement of volumetric flow, J. Ultrasound Med. vol. 25, at pp 1305-1311 (2006). See also US Pat. 6,780,155 (Li). Since the coronal plane intersects the whole vessel, there is no assumption of uniform insonation and also no assumption about the vessel being circular. Also, neither the Doppler angle or the vessel diameter need to be measured, since the ultrasound beams transmitted are perpendicular to each point on the surface. FIGURE 2a illustrates a vessel 70 which is intersected by a Gaussian surface 50. The thin plane 52 of the surface is scanned by the transmission of Doppler beams from a 2D array transducer 54, which electronically steers the beams over the surface 50 where it intersects the vessel 70. A flow image 76 is thereby rendered as a cross-sectional surface of the curved cross-section 58 through the vessel 70. As explained in the foregoing '155 patent, the flow image 76 can be projected onto a planar surface 72 as a B mode image 56. The vessel lumen 62 in the B mode image can be segmented by a circle 64 or other shape outside the vessel wall 60, and color voxels within the segmented area are then summed to produce an estimate of volume flow. Corrections should be made for color voxels at the vessel walls which may only have partial flow, and one way to do this is through normalization of the velocity estimates using the power (intensity) in the Doppler signal (see Kripfgans *et al.*, above), often known as partial volume correction.

While this is an excellent method for measuring volume flow, there are challenges in measuring pulsatile flow due to the typically limited volume rates possible with 3D/4D color Doppler, resulting in under-sampled temporal information and erroneous flow volume calculation. Each point on the Gaussian surface must be sampled by an individual Doppler beam, and multiple times by multiple transmissions so as to estimate the Doppler velocity at each point on the Gaussian surface accurately. To mitigate this limitation related methods have been developed to acquire information over multiple cardiac cycles, and then either averaged to get average volume flow or, if the cardiac period is precisely known, it is possible to reconstruct a single cardiac cycle from the multiple cycles. However, these approaches add to the acquisition time and make the method less robust due to the temporal sampling time required.

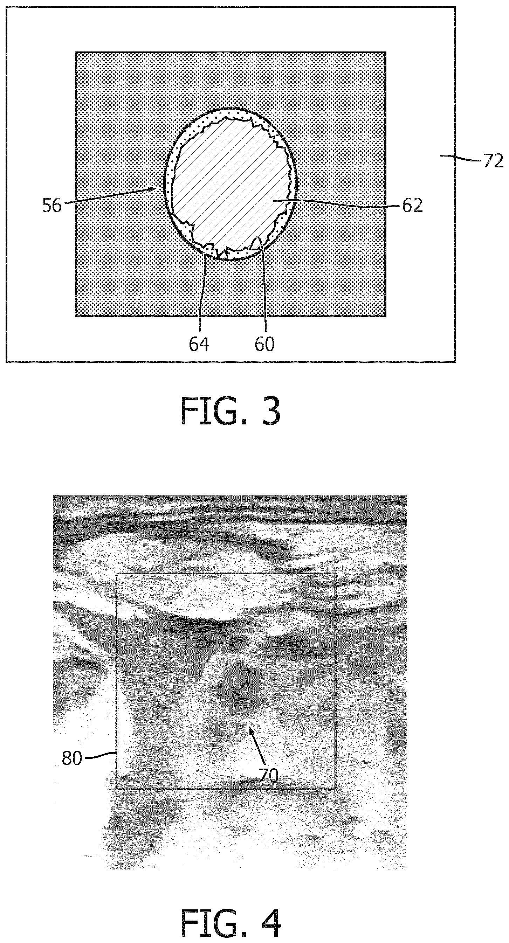

Another method with similarities to the Gaussian surface integration method has been proposed by Picot *et al.* See Picot et al., Rapid volume flow rate estimation using transverse colour Doppler imaging, Ultrasound in Medicine and Biology, vol. 21 (9), at pp 1199-1209 (1995). In this method, instead of extracting a coronal plane from a 3D color Doppler volume, a conventional 1D array transducer is angled toward a vessel so that its scan plane, and 2D color image, intersects the vessel at an oblique but transverse angle. FIGURE 4 illustrates an ultrasound image of a vessel 70 scanned in this manner within a color box 80. Similar to the Gaussian surface integration method, all the color Doppler pixels in the color box 80 are summed, with corrections applied for partially filled pixels at the edges of the vessel. Again, there is no assumption about flow profile or vessel geometry. One major benefit compared to the Gaussian surface integration method is that two-dimensional color frame rates are typically much higher than 3D/4D color volume rates, so adequate temporal sampling for pulsatile flow is much improved. However, one disadvantage compared to the Gaussian surface integration method is that the Doppler angle must be known, which is very difficult to obtain from a transverse image. Picot *et al.* describe an elaborate probe holder that allows the same vessel to be interrogated from two angles, allowing the Doppler angle dependency to be eliminated, but such a probe holder is cumbersome and impractical for clinical use.

In accordance with the principles of the present disclosure, an ultrasound probe with a two dimensional matrix array transducer is operated in the biplane mode to measure volume flow. In the biplane mode, two image planes are scanned simultaneously in an interleaved manner. While the biplane mode can be performed by a mechanical probe which moves a 1D transducer array to scan two image planes of a volumetric region as described in US Pat. 6,443,896 (Detmer), it is preferable to use a 2D matrix array probe by which the planes are scanned electronically, rather than mechanically, as described in US Pat. 6,709,394 (Frisa et al.) Furthermore, it is possible to perform colorflow imaging in the biplane mode as described in US Pat. 7,645,237 (Frisa et al.) whereby a color box is scanned to acquire color Doppler data in each of the biplane image planes. Ultrasound systems and probes are commercially available which can perform colorflow scanning of biplane images, such as the xMATRIX family of probes available on Philips Healthcare ultrasound systems. In one implementation of the present disclosure the biplane mode of scanning is performed to generate two real-time images, including color Doppler data, that are perpendicular to each other. This allows the production, simultaneously, of a long axis view of a vessel and a transverse view. The long axis image can be used to accurately measure the Doppler angle, as described below. The transverse color image intersects the vessel obliquely, in the same manner as in Picot's method, so the same algorithm can be used to estimate the volume flow by summing all the color pixels, but with an accurately known Doppler angle correction obtained from the long axis image.

An implementation of the present disclosure overcomes many of the limitations and shortcomings of the prior methods for measuring volume flow. As compared to the pulsed Doppler method, an implementation of the present disclosure requires no assumptions of uniform insonation or vessel geometry, and there is no need to measure the vessel diameter, which is the greatest cause of inaccuracy in the typical pulsed Doppler-based method. As compared to the Gaussian surface integration method, the inventive technique provides much better temporal sampling since only two image planes need be scanned and so is more suitable for very pulsatile flow as would be found in many arteries. In addition, spatial sampling is uncompromised, as there is no need to try to improve 3D volume frame rates. Better spatial sampling results in better representation of the flow profile and also reduced reliance on partial volume correction. As compared to the method of Picot *et al.,* an implementation of the present disclosure makes it very easy to accurately measure the Doppler angle needed for velocity correction.

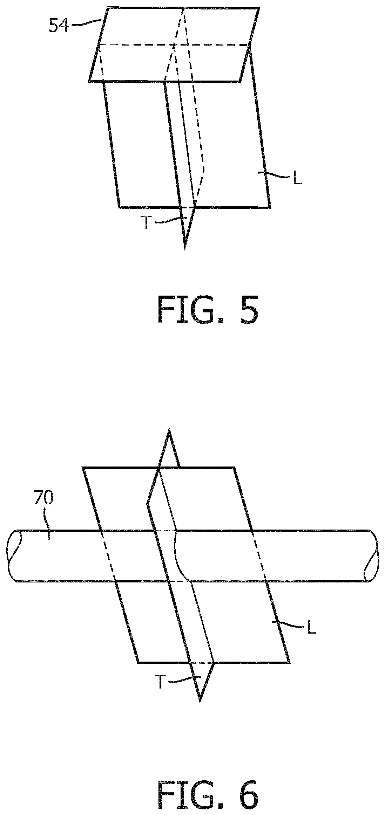

The operation and use of an implementation of the present disclosure may be appreciated by referring to FIGURES 5 and 6. FIGURE 5 depicts a 2D matrix array transducer 54 which scans two biplanes in front of the transducer, denoted L and T. When all of the scanlines transmitted and received for a plane are normal to the plane of the 2D array transducer, the plane will extend normal to the transducer as shown in this illustration. When the scanlines are transmitted and received at an oblique angle to the plane of the array transducer, the scan plane will be angled in the shape of a parallelogram, which results from steered linear operation. In the example of FIGURE 5 the two biplanes extend normal to the transducer, as the scanlines are transmitted and received straight ahead of the array. The L and T planes are seen to intersect at a common intermediate scanline.

FIGURE 6 shows the two L and T biplanes being steered to intersect and scan a blood vessel 70 in accordance with the present disclosure. The L plane is tilted from the upper left to the lower right and is aimed by probe manipulation at the longitudinal center of the vessel 70, thereby producing a long axis view of the vessel. The L scan plane is tilted in a parallelogram orientation so that the scanlines of the plane will intersect the direction of blood flow of the vessel at a nonorthogonal angle since, as is well known, a 90° angle between a Doppler beam and the flow direction will yield no measurable Doppler signal since the cosine of 90° is zero. The T plane is aligned with one of the parallel scanlines of the L plane and intersects the blood vessel 70 obliquely, thereby producing a transverse view of the vessel where the T image plane cuts through the vessel. The Doppler angle of the T plane which is needed for angle correction of the Doppler velocity measurements is thus the angle at which the L plane is tilted, which is readily known from the Doppler angle of the L plane and the readily observable flow direction in the long axis view of the vessel.

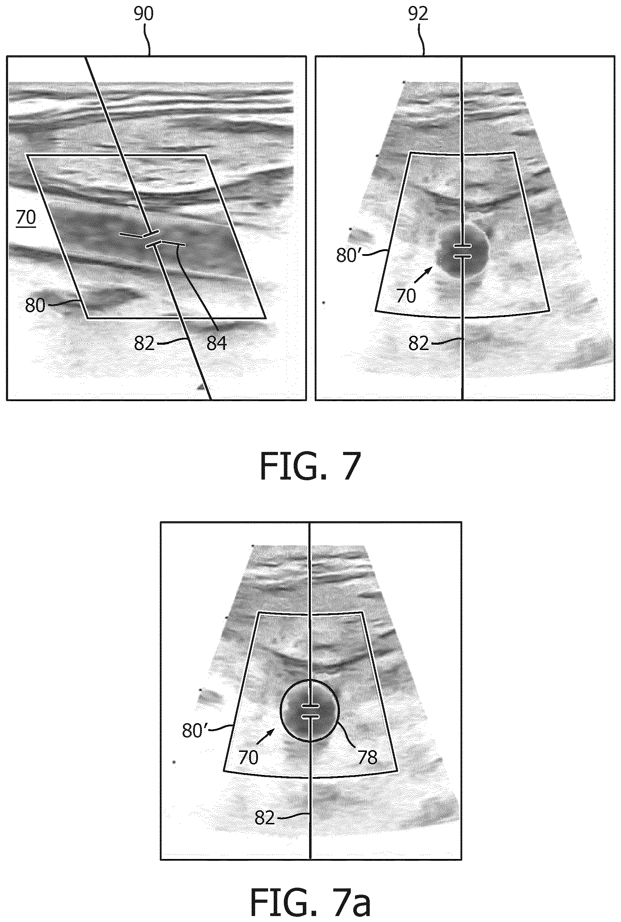

When a biplane probe is used to scan a vessel as illustrated in FIGURE 6, long axis and transverse views of the vessel can be produced and displayed simultaneously as illustrated in FIGURE 7. In this example the biplane images are shown side-by-side in a duplex display. The left image 90 is the long axis view of the L scan plane of FIGURE 6, showing vessel 70 in a long axis view bisecting the vessel. Within the grayscale (B mode) image of the blood vessel and surrounding tissue is a color box 80 which is scanned by Doppler beams for Doppler display of the material inside the box. Like the color box of FIGURE 1, the color box 80 is tilted at an angle as established by the setting of the tilt angle of the Doppler gate line 82. Like the previous drawing, the Doppler line has a flow cursor 84, which the user aligns with the direction of the flow in vessel 70. The angle between the Doppler gate line 82 and the flow cursor 84 establishes the Doppler angle, the angle between the Doppler beams used to scan the color box 80 and the direction of the blood flow. The Doppler angle is commonly recognized and recorded automatically in standard ultrasound systems.

In an implementation of the present disclosure the image 92 of the transverse view of the vessel 90 is scanned in a plane in alignment with the angle of the color box 80. Typically, the image planes 90 and 92 are spatially normal to each other. In this example the plane of the image 92 is in alignment with the Doppler gate line 82 of image 90; the two images spatially share a common location of their Doppler lines 82. The result is that the Doppler angle correction needed for the velocity values of flow in the transverse image 92 is the Doppler angle of the longitudinal view 90, the angle between the Doppler gate line 82 and the flow cursor 84, which is readily recognized in a typical commercial ultrasound system. The ultrasound system can then measure volume flow by any of several known algorithms such as that of Picot *et al.,* in which the color pixel values of the vessel in the transverse view are angle-corrected, then summed to compute volume flow. Mathematically, this can be represented by Gauss's theorem, calculated as:

$$Q = \int_S v \cdot \mathrm{d}A$$

where Q is the volume flow in, *e.g.*, milliliters per second, v is angle-corrected flow velocity, and the surface S is the Doppler portion of the cut plane through the vessel lumen in the transverse view 92. In addition, a typical commercial ultrasound system will enable a user to segment (delineate) the portion of the image over which Doppler velocity pixels are to be integrated. FIGURE 7a presents an example of such a tool, a circle template 78 which a user can appropriately size and then maneuver over an ultrasound image to designate the image area within which Doppler value integration is to occur. Such segmentation of the vessel lumen will prevent the volume flow algorithm from mistakenly including pixel values of neighboring blood vessels, for instance.

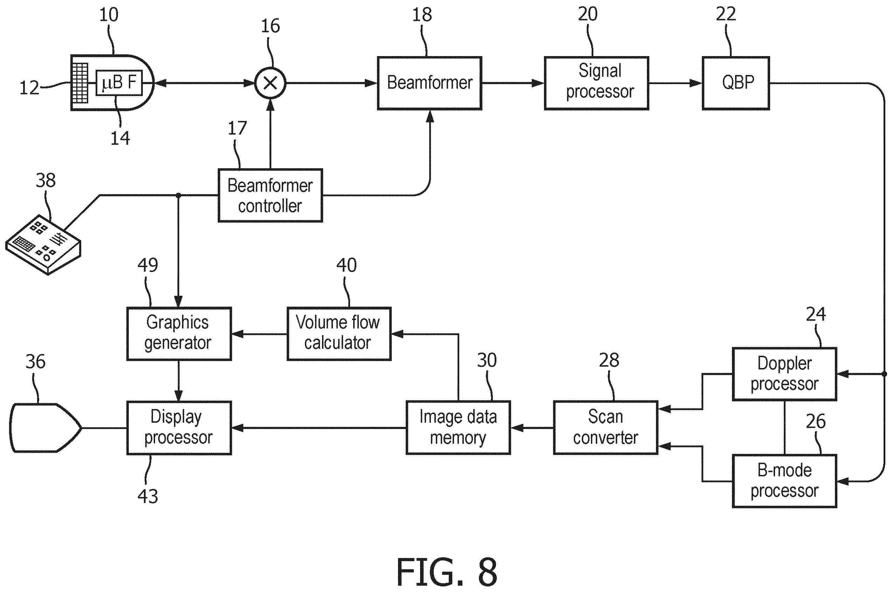

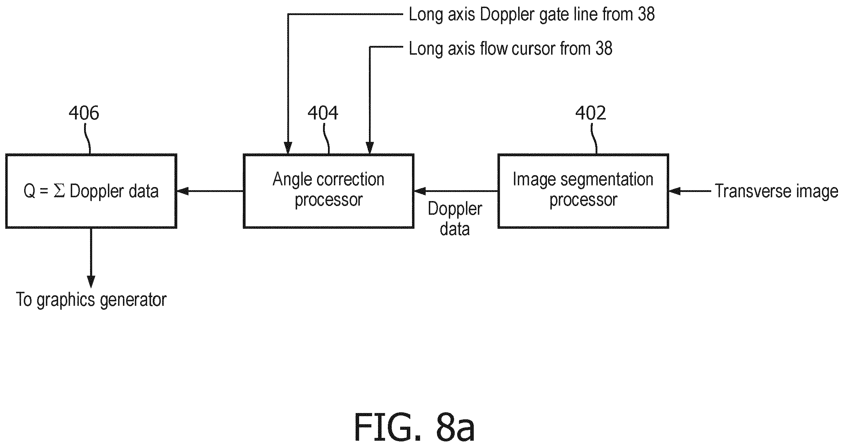

In FIGURE 8, an ultrasound system constructed in accordance with the principles of the present disclosure is shown in block diagram form. A transducer array 12 is provided in an ultrasound probe 10 for transmitting ultrasonic waves and receiving echo information. The transducer array 12 is a two-dimensional array of transducer elements capable of scanning in three dimensions, in both elevation and azimuth. It is thus capable of scanning two biplanes simultaneously in a time-interleaved manner. The transducer array 12 is coupled to a microbeamformer 14 in the probe which controls transmission and reception of signals by the array elements. Microbeamformers are capable of at least partial beamforming of the signals received by groups or "patches" of transducer elements as described in US Pats. 5,997,479 (Savord et al.), 6,013,032 (Savord), and 6,623,432 (Powers et al.) The microbeamformer is coupled by the probe cable to a transmit/receive (T/R) switch 16 which switches between transmission and reception and protects the main beamformer 18 from high energy transmit signals. The transmission of ultrasonic beams from the transducer array 12 under control of the microbeamformer 14 is directed by a beamformer controller 17 coupled to the T/R switch and the main beamformer 18, which receives input from the user's operation of the user interface or control panel 38. Among the transmit characteristics controlled by the transmit controller are the direction, number, spacing, amplitude, phase, angle, frequency, polarity, and diversity of transmit waveforms. Beams formed in the direction of pulse transmission may be steered straight ahead from the transducer array, or at different angles on either side of an unsteered beam for a wider sector field of view, or for transmission at a selected Doppler angle.

The echoes received by a contiguous group of transducer elements are beamformed by appropriately delaying them and then combining them. The partially beamformed signals produced by the microbeamformer 14 from each patch are coupled to the main beamformer 18 where partially beamformed signals from individual patches of transducer elements are combined into a fully beamformed coherent echo signal. For example, the main beamformer 18 may have 128 channels, each of which receives a partially beamformed signal from a patch of 12 transducer elements. In this way the signals received by over 1500 transducer elements of a two-dimensional matrix array transducer can contribute efficiently to a single beamformed signal.

The coherent echo signals undergo signal processing by a signal processor 20, which includes filtering by a digital filter and noise and speckle reduction as by spatial or frequency compounding. The digital filter of the signal processor 20 can be a filter of the type disclosed in U.S. Patent No. 5,833,613 (Averkiou et al.), for example. The echo signals are then coupled to a quadrature bandpass filter (QBP) 22. The QBP performs three functions: band limiting the r.f. echo signal data, producing in-phase and quadrature pairs (I and Q) of echo signal data, and decimating the digital sample rate. The QBP comprises two separate filters, one producing in-phase samples and the other producing quadrature samples, with each filter being formed by a plurality of multiplier-accumulators (MACs) implementing an FIR filter.

The beamformed and processed coherent echo signals are coupled to a pair of image data processors. A B mode processor 26 produces signal data for a B mode image of structure in the body such as tissue and blood vessel walls. The B mode processor performs amplitude (envelope) detection of quadrature demodulated I and Q signal components by calculating the echo signal amplitude in the form of $(I^2+Q^2)^{1/2}$. The quadrature echo signal components are also coupled to a Doppler processor 24. The Doppler processor 24 stores ensembles of echo signals from discrete points in an image field which are then used to estimate the Doppler shift at points in the image with a fast Fourier transform (FFT) processor. The Doppler processor can also perform angle correction of Doppler velocity values, and in an implementation of the present invention angle correction as measured on a first (long axis) Doppler image is used to perform angle correction of the Doppler data of a second (transverse) Doppler image used to determine volume flow. The rate at which the ensembles are acquired determines the velocity range of motion that the system can accurately measure and depict in an image. The Doppler shift is proportional to motion at points in the image field, *e.g.*, blood flow and tissue motion. For color Doppler image data, the estimated Doppler flow values at each point in a blood vessel are wall filtered, angle corrected, and converted to color values using a look-up table. The wall filter has an adjustable cutoff frequency above or below which motion will be rejected such as the low frequency motion of the wall of a blood vessel when imaging flowing blood. The B mode image data and the Doppler flow values are coupled to a scan converter 28 which converts the B mode and Doppler samples from their acquired R-θ coordinates to Cartesian (x,y) coordinates for display in a desired display format, *e.g.*, a rectilinear display format or a sector display format as shown in FIGURES 7 and 7a. Either the B mode image or the Doppler image may be displayed alone, or the two shown together in anatomical registration in which the color Doppler overlay shows the blood flow in B mode processed tissue and vessels in the image as shown in FIGURES 7 and 7a. Another display possibility is to display side-by-side images of the same anatomy which have been processed differently, as shown in these drawings. This side-by-side display format is useful when comparing images, and is particularly useful for displaying both images of a biplane probe. The scan-converted image data, both B mode and Doppler data, is coupled to and stored in an image data memory 30 where it is stored in memory locations addressable in accordance with the spatial locations from which the image data values were acquired. The biplane images from data produced by the scan converter 28 and stored in the image data memory are coupled to a display processor 34 for further enhancement, buffering and temporary storage for display on an image display 36.

The Doppler values of an image, such as the color Doppler pixel values of a transverse view of a blood vessel as shown in image 92 of FIGURE 7, are coupled to a volume flow calculator 40. There, an algorithm computing volume flow is executed, such as the integration of pixel flow velocity values over the area of a vessel lumen as exemplified by the expression

$$Q = \int_S v \cdot \mathrm{d}A$$

Volume flow may be calculated for each frame separately in the color Doppler image to provide volume flow as a function of time, or may be summed over multiple frames to provide a time-average volume flow rate. The volume flow measurement is coupled to a graphics generator 49, from which the volume flow value is coupled to the display processor 34 for display in conjunction with the ultrasound images. Alternatively or additionally, the graphics generator 49 can produces a flow profile curve for display on the display 36. The graphics generator also produces graphics for display with the ultrasound image for things such as cursors, measurement dimensions, exam parameters, patient name, and the aforementioned Doppler gate line 82, flow cursor 84, and segmentation template 78.

Details of the operation of the volume flow calculator 40 of FIGURE 8 are illustrated in FIGURE 8a. In this implementation, angle correction of the transverse image 92 is performed by the volume flow calculator rather than the Doppler processor 24. An image segmentation processor 402 receives the data of transverse image 92 from the image data memory 30. The Doppler data in the transverse view of vessel 70 is identified (segmented) by the placement by the system operator of a template such as a circle template 78 over the lumen of vessel 70. The Doppler data thereby designated within the vessel 70 is coupled to an angle correction processor 404, which computes the angle correction performed for Doppler data of the long axis view 90 from the long axis Doppler gate line 82 and the long axis flow cursor 84, which is the angle between those two graphics as set by the user during acquisition of the long axis view 90. The Doppler values of the flow in the transverse image are thereby angle corrected in accordance with the angle set by these graphics during acquisition of the long axis view 90. The angle-corrected Doppler values are then integrated over the vessel area in the transverse image 92 to determine the volume flow. In the implementation of FIGURE 8a this operation is performed by summing the angle-corrected Doppler flow values of the transverse view image as illustrated by processor 406 to produce a measure Q of volume flow. This value is coupled to the graphics generator 49 for display to the ultrasound system operator. In a typical implementation the computation performed by the processors 402, 404, and 406 are performed by software programs configured to perform the illustrated functions.

The ultrasound system of FIGURE 8 can be operated to perform a typical ultrasound carotid artery exam as follows. The user would first examine the carotid artery by ultrasound imaging, including B-mode, color Doppler and pulsed Doppler imaging in accordance with standard clinical practice. On identifying a significant stenosis, the user then takes the following steps to determine the volume flow. First, scan with the ultrasound probe to find a section of the artery upstream of the stenosis to minimize turbulence in the blood flow. Next, biplane mode is actuated. The probe is manipulated until the vessel appears stretched out in a long axis view on the left side image 90. The probe is tilted in elevation until the vessel is roughly in the center of the right side image 92. The color scale (pulse repetition frequency, PRF) is adjusted so that the flow is not aliased. The angle correction is adjusted so that it aligns with the vessel 70 in the left side image 90. One or more cardiac cycles of flow information are then acquired. A region of interest is designated (segmented) by placing a template 78 over the vessel 70 in the right side image to define which color pixels in the image should be included in the volume flow calculation. Volume flow is then calculated and displayed using Doppler data of the transverse image as angle-corrected from the angle correction of the long axis view, either as an average over time (a single number) or as a graph of volume flow as a function of time.

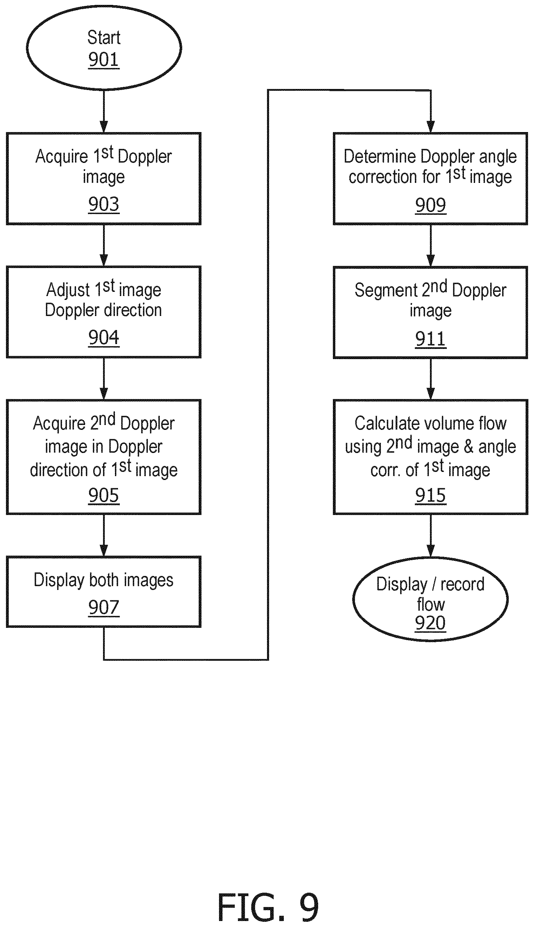

A method for measuring volume flow in accordance with the principles of the present disclosure may be conducted as shown in FIGURE 9. At the start 901, an ultrasound system is set to operate in the biplane mode, acquiring two images which intersect at a Doppler angle of one of them. At 903, a first Doppler image is acquired such as the long axis view 90 of FIGURE 7. The direction of Doppler acquisition of this first image is adjusted at 904 by adjusting a Doppler gate line 82. After the Doppler direction has been set, a second Doppler image is acquired at 905 at a plane in the Doppler line direction of the first image. At 907 both images are simultaneously displayed. The Doppler angle correction for the first image is determined at 909 from the Doppler line direction and the direction of a flow cursor set by the user over the flow of the first image. At 911 the flow of the second Doppler image is segmented as by placing a graphic circle template over the lumen of the second image. At 915 volume flow is calculated as described above from the Doppler values of the flow of the second image as angle-corrected by the angle correction determine for the first image. At 920 the measured volume flow is displayed to the user or recorded in the ultrasound exam record.

It is noted that the scope of the invention described above also includes embodiments which do not necessarily include an ultrasound probe, but which instead receives an input of acquired Doppler image data from two image planes (90, 92) intersecting along a Doppler beam direction and adapted to produce two Doppler images of flow. The invention further includes a display (36) adapted to display the two Doppler images simultaneously, and a graphics generator (49), responsive to a user control, and adapted to display a Doppler line (82) and a flow cursor (84) over a first one (90) of the Doppler images. A volume flow calculator (40) is responsive to Doppler image data of the second one (92) of the Doppler images and a Doppler angle established by the Doppler line and the flow cursor, which is adapted to determine an angle-corrected measure of volume flow.

It should further be noted that an ultrasound system suitable for use in an implementation of the present disclosure, and in particular the component structure of the ultrasound system of FIGURE 8, may be implemented in hardware, software or a combination thereof. The various embodiments and/or components of an ultrasound system and its controller, or components and controllers therein, also may be implemented as part of one or more computers or microprocessors. The computer or processor may include a computing device, an input device, a display unit and an interface, for example, for accessing the internet. The computer or processor may include a microprocessor. The microprocessor may be connected to a communication bus, for example, to access a PACS system or the data network for importing training images and storing the results of clinical exams. The computer or processor may also include a memory. The memory devices such as the image data memory may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or instructions for volume flow analysis into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine. The set of instructions of an ultrasound system including those controlling the acquisition, processing, and display of ultrasound images and instructions for Doppler angle measurement and volume flow calculation as described above may include various commands that instruct a computer or processor as a processing machine to perform specific operations such as the methods and processes of Doppler flow data acquisition, line and cursor adjustment, and volume flow measurement. The set of instructions may be in the form of a software program. The software may be in various forms such as system software or application software and which may be embodied as a tangible and non-transitory computer readable medium. The equation given above for volume flow calculation and the summation of Doppler data values shown in FIGURE 8a, as well as the calculation of the Doppler angle from the cursors placed over an image, are typically calculated by or under the direction of software routines. Further, the software may be in the form of a collection of separate programs or modules within a larger program or a portion of a program module. The software also may include modular programming in the form of object-oriented programming. The processing of input data by the processing machine may be in response to operator commands issued from control panel 38, or in response to results of previous processing, or in response to a request made by another processing machine.

**Claims**

1. An ultrasonic diagnostic imaging system for analyzing volume flow of blood comprising:

   an ultrasound probe (10) comprising a two-dimensional matrix array transducer adapted to acquire Doppler image data from two image planes (90, 92) intersecting along a Doppler beam direction, wherein the ultrasound probe is further adapted to simultaneously acquire a long axis view of a vessel and a transverse view of the vessel;
   an image data processor (24) responsive to the acquired Doppler image data and adapted to produce two Doppler images of flow, wherein the first image is produced responsive to the image data acquired in the long axis view of the vessel and the second image is produced responsive to the image data acquired in the transverse view of the vessel;
   a display (36) adapted to display the two Doppler images simultaneously;
   a graphics generator (49), responsive to a user control, and adapted to display a Doppler line (82) and a flow cursor (84) over a first one (90) of the Doppler images; and
   a volume flow calculator (40), responsive to Doppler image data of the second one (92) of the Doppler images and a Doppler angle established by the Doppler line and the flow cursor, which is adapted to determine an angle-corrected measure of the volume flow.

2. The ultrasonic diagnostic imaging system of Claim 1, wherein the Doppler line is aligned with the direction of a Doppler beam which scans the first image; and wherein the image plane of the second image is aligned with the direction of the Doppler beam of the first image.

3. The ultrasonic diagnostic imaging system of Claim 1, wherein the first image is acquired by scanning an image plane with a plurality of parallel Doppler beams transmitted in a given direction, wherein the Doppler line is aligned with the direction of the Doppler beams.

4. The ultrasonic diagnostic imaging system of Claim 3, wherein the image plane of the second image is aligned with the direction of the Doppler beams of the first image.

5. The ultrasonic diagnostic imaging system of Claim 1, wherein the ultrasound probe is further adapted to operate in a biplane mode to scan two image planes in a time-interleaved manner.

**6.** The ultrasonic diagnostic imaging system of Claim 5, wherein the ultrasound probe is further adapted to scan two image planes at a selected nonorthogonal angle to the plane of the matrix array transducer.

**7.** The ultrasonic diagnostic imaging system of Claim 5, wherein the ultrasound probe is further adapted to scan an image plane at a selected nonorthogonal angle to a direction of flow.

**8.** The ultrasonic diagnostic imaging system of Claim 1, wherein the volume flow calculator is further adapted to calculate an algorithm of the form

$$Q = \int_S v \cdot dA$$

where Q is the volume flow, v is the angle-corrected flow velocity, surface S is a cut plane through a vessel containing Doppler data and A is the area of the vessel lumen.

**9.** The ultrasonic diagnostic imaging system of Claim 8, wherein the volume flow calculator is further adapted to sum values of angle-corrected Doppler data within a vessel lumen.

**10.** A method of using an ultrasonic diagnostic imaging system to conduct an ultrasound exam to measure volume flow comprising:

scanning (903) with an ultrasound probe, the probe comprising a two-dimensional matrix array transducer, to simultaneously acquire Doppler image data from two image planes (90, 92) intersecting along a Doppler beam direction and producing from the Doppler image data two Doppler images of flow, wherein the first Doppler image is of a target vessel in a long axis view and the second Doppler image is of the target vessel in a transverse view;
displaying (907) the two images simultaneously;
responsive to a user control, displaying a Doppler line (82) and a flow cursor (84) over a first one (90) of the Doppler images;
determining angle correction (909) in accordance with a Doppler beam direction and a flow direction of the first Doppler image established by the Doppler line and the flow cursor; and
calculating the volume flow (915) from data of the second Doppler image using angle correction determined from the first Doppler image.

**11.** The method of claim 10, wherein the probe is adapted to operate in a biplane mode to acquire the first Doppler image and the second Doppler image.

**12.** The method of claim 10 or 11, wherein the second Doppler image is in an image plane aligned with a Doppler angle of the first Doppler image.

**13.** The method of Claim 10, further comprising adjusting the Doppler beam direction for the first Doppler image to intersect the flow direction at a nonorthogonal angle, and

optionally further comprising segmenting the flow in the second Doppler image with a template, and
optionally wherein calculating volume flow further comprises integrating flow value pixels of the target vessel in the second image.

**14.** An ultrasonic diagnostic imaging system for analyzing volume flow of blood comprising:

an image data processor (24) responsive to an input from an ultrasound probe comprising a two-dimensional matrix array transducer and adapted to produce two Doppler images of flow, said input comprising Doppler image data from two image planes (90, 92) intersecting along a Doppler beam direction acquired by the ultrasound probe simultaneously, wherein a first of the two images has a long axis view of a vessel, and the second of the two images has a transverse view of the vessel;
a display (36) adapted to display the two Doppler images simultaneously;
a graphics generator (49), responsive to a user control, and adapted to display a Doppler line (82) and a flow cursor (84) over a first one (90) of the Doppler images; and
a volume flow calculator (40), responsive to Doppler image data of the second one (92) of the Doppler images and a Doppler angle established by the Doppler line and the flow cursor, which is adapted to determine an angle-corrected measure of the volume flow.

**Patentansprüche**

**1.** Ultraschall-Diagnosebildgebungssystem zum Analysieren des Volumenflusses von Blut, umfassend:

eine Ultraschallsonde (10), die einen zweidimensionalen Matrixarray-Wandler umfasst, der dazu geeignet ist, Doppler-Bilddaten aus zwei Bildebenen (90, 92) zu erfassen, die sich entlang einer Doppler-Strahlrichtung schneiden, wobei die Ultraschallsonde weiter dazu geeignet ist, gleichzeitig eine Längsachsenansicht eines Gefäßes und eine Queransicht des Gefäßes zu erfassen;

einen Bilddatenprozessor (24), der auf die erfassten Doppler-Bilddaten reagiert und dazu geeignet ist, zwei Doppler-Bilder eines Flusses zu erzeugen, wobei das erste Bild als Reaktion auf die in der Längsachsenansicht des Gefäßes erfassten Bilddaten erzeugt wird und das zweite Bild als Reaktion auf die in der Queransicht des Gefäßes erfassten Bilddaten erzeugt wird;

eine Anzeige (36), die dazu geeignet ist, die zwei Doppler-Bilder gleichzeitig anzuzeigen;

einen Grafikgenerator (49), der auf eine Benutzersteuerung reagiert und dazu geeignet ist, eine Doppler-Linie (82) und einen Flusszeiger (84) über einem ersten (90) der Doppler-Bilder anzuzeigen; und

einen Volumenflussrechner (40), der auf Doppler-Bilddaten des zweiten (92) der Doppler-Bilder und einen durch die Doppler-Linie und den Flusszeiger festgelegten Doppler-Winkel reagiert und dazu geeignet ist, ein winkelkorrigiertes Maß für den Volumenfluss zu bestimmen.

2. Ultraschall-Diagnosebildgebungssystem nach Anspruch 1, wobei die Doppler-Linie mit der Richtung eines Doppler-Strahls ausgerichtet ist, der das erste Bild abtastet; und

wobei die Bildebene des zweiten Bilds mit der Richtung des Doppler-Strahls des ersten Bilds ausgerichtet ist.

3. Ultraschall-Diagnosebildgebungssystem nach Anspruch 1, wobei das erste Bild durch Abtasten einer Bildebene mit einer Vielzahl von parallelen Doppler-Strahlen erfasst wird, die in einer vorgegebenen Richtung ausgestrahlt werden, wobei die Doppler-Linie mit der Richtung der Doppler-Strahlen ausgerichtet ist.

4. Ultraschall-Diagnosebildgebungssystem nach Anspruch 3, wobei die Bildebene des zweiten Bilds mit der Richtung der Doppler-Strahlen des ersten Bilds ausgerichtet ist.

5. Ultraschall-Diagnosebildgebungssystem nach Anspruch 1, wobei die Ultraschallsonde weiter dazu geeignet ist, in einem Zwei-Ebenen-Modus zu arbeiten, um zwei Bildebenen zeitversetzt abzutasten.

6. Ultraschall-Diagnosebildgebungssystem nach Anspruch 5, wobei die Ultraschallsonde weiter dazu geeignet ist, zwei Bildebenen in einem ausgewählten nichtorthogonalen Winkel zu der Ebene des Matrixarray-Wandlers abzutasten.

7. Ultraschall-Diagnosebildgebungssystem nach Anspruch 5, wobei die Ultraschallsonde weiter dazu geeignet ist, eine Bildebene in einem ausgewählten nichtorthogonalen Winkel zu einer Flussrichtung abzutasten.

8. Ultraschall-Diagnosebildgebungssystem nach Anspruch 1, wobei der Volumenflussrechner weiter dazu geeignet ist, einen Algorithmus der folgenden Formel zu berechnen

$$Q = \int_S v \cdot \mathrm{d}A$$

wo Q der Volumenfluss ist, v die winkelkorrigierte Flussgeschwindigkeit ist, Oberfläche S eine Schnittebene durch ein Gefäß ist, das Doppler-Daten enthält, und A die Fläche des Gefäßlumens ist.

9. Ultraschall-Diagnosebildgebungssystem nach Anspruch 8, wobei der Volumenflussrechner weiter dazu geeignet ist, Werte von winkelkorrigierten Doppler-Daten innerhalb eines Gefäßlumens zu summieren.

10. Verfahren zur Verwendung eines Ultraschall-Diagnosebildgebungssystems zum Durchführen einer Ultraschalluntersuchung, um einen Volumenfluss zu messen, umfassend:

Abtasten (903) mit einer Ultraschallsonde, wobei die Sonde einen zweidimensionalen Matrixarray-Wandler umfasst, um gleichzeitig Doppler-Bilddaten von zwei Bildebenen (90, 92) zu erfassen, die sich entlang einer Doppler-Strahlrichtung schneiden, und um aus den Doppler-Bilddaten zwei Doppler-Bilder des Flusses zu erzeugen, wobei das erste Doppler-Bild ein Zielgefäß in einer Längsachsenansicht zeigt und das zweite Doppler-Bild das Zielgefäß in einer Queransicht zeigt;

gleichzeitiges Anzeigen (907) der zwei Bilder;

als Reaktion auf eine Benutzersteuerung, Anzeigen einer Doppler-Linie (82) und eines Flusszeigers (84) über einem ersten (90) der Doppler-Bilder;

Bestimmen einer Winkelkorrektur (909) in Übereinstimmung mit einer Doppler-Strahlrichtung und einer Flussrichtung des ersten Doppler-Bilds, das durch die Doppler-Linie und den Flusszeiger festgelegt wird; und

Berechnen des Volumenflusses (915) aus Daten des zweiten Doppler-Bilds unter Verwendung einer aus dem ersten Doppler-Bild ermittelten Winkelkorrektur.

11. Verfahren nach Anspruch 10, wobei die Sonde dazu geeignet ist, in einem Zwei-Ebenen-Modus zu arbeiten, um das erste Doppler-Bild und das zweite Doppler-Bild zu erfassen.

**12.** Verfahren nach Anspruch 10 oder 11, wobei sich das zweite Doppler-Bild in einer Bildebene befindet, die mit einem Doppler-Winkel des ersten Doppler-Bilds ausgerichtet ist.

**13.** Verfahren nach Anspruch 10, das weiter Anpassen der Doppler-Strahlrichtung für das erste Doppler-Bild umfasst, um die Flussrichtung in einem nichtorthogonalen Winkel zu schneiden, und

> optional weiter Segmentieren des Flusses in dem zweiten Doppler-Bild mit einer Schablone umfasst, und
> wobei optional Berechnen des Volumenflusses weiter Integrieren von Flusswertpixeln des Zielgefäßes in dem zweiten Bild umfasst.

**14.** Ultraschall-Diagnosebildgebungssystem zum Analysieren des Volumenflusses von Blut, umfassend:

> einen Bilddatenprozessor (24), der auf eine Eingabe von einer Ultraschallsonde reagiert, die einen zweidimensionalen Matrixarray-Wandler umfasst und dazu ausgelegt ist, zwei Doppler-Bilder des Flusses zu erzeugen, wobei die Eingabe Doppler-Bilddaten von zwei Bildebenen (90, 92) umfasst, die sich entlang einer Doppler-Strahlrichtung schneiden und gleichzeitig von der Ultraschallsonde erfasst werden,
> wobei ein erstes der zwei Bilder eine Längsachsenansicht eines Gefäßes zeigt und das zweite der zwei Bilder eine Queransicht des Gefäßes zeigt;
> eine Anzeige (36), die dazu geeignet ist, die zwei Doppler-Bilder gleichzeitig anzuzeigen;
> einen Grafikgenerator (49), der auf eine Benutzersteuerung reagiert und dazu geeignet ist, eine Doppler-Linie (82) und einen Flusszeiger (84) über einem ersten (90) der Doppler-Bilder anzuzeigen; und
> einen Volumenflussrechner (40), der auf Doppler-Bilddaten des zweiten (92) der Doppler-Bilder und einen durch die Doppler-Linie und den Flusszeiger festgelegten Doppler-Winkel reagiert und dazu geeignet ist, ein winkelkorrigiertes Maß für den Volumenfluss zu bestimmen.

**Revendications**

**1.** Système d'imagerie diagnostique ultrasonore pour analyser un débit volumique du sang comprenant :

> une sonde à ultrasons (10) comprenant un transducteur à réseau matriciel bidimensionnel conçu pour acquérir des données d'image Doppler à partir de deux plans d'image (90, 92) se croisant le long d'une direction de faisceau Doppler, dans lequel la sonde à ultrasons est en outre conçue pour acquérir simultanément une vue d'axe long d'un vaisseau et une vue transversale du vaisseau ;
> un processeur de données d'image (24) sensible aux données d'image Doppler acquises et conçu pour produire deux images Doppler de débit, dans lequel la première image est produite en réponse aux données d'image acquises dans la vue d'axe long du vaisseau et la seconde image est produite en réponse aux données d'image acquises dans la vue transversale du vaisseau ;
> un écran (36) conçu pour afficher les deux images Doppler simultanément ;
> un générateur graphique (49), sensible à une commande utilisateur, et conçu pour afficher une ligne Doppler (82) et un curseur de débit (84) sur une première image (90) des images Doppler ; et
> un calculateur de débit volumique (40), sensible aux données d'image Doppler de la seconde image (92) des images Doppler et à un angle Doppler établi par la ligne Doppler et le curseur de débit, qui est conçu pour déterminer une mesure à angle corrigé du débit volumique.

**2.** Système d'imagerie diagnostique ultrasonore selon la revendication 1, dans lequel la ligne Doppler est alignée avec la direction d'un faisceau Doppler qui balaie la première image ; et
dans lequel le plan d'image de la seconde image est aligné avec la direction du faisceau Doppler de la première image.

**3.** Système d'imagerie diagnostique ultrasonore selon la revendication 1, dans lequel la première image est acquise en balayant un plan d'image avec une pluralité de faisceaux Doppler parallèles émis dans une direction donnée, dans lequel la ligne Doppler est alignée avec la direction des faisceaux Doppler.

**4.** Système d'imagerie diagnostique ultrasonore selon la revendication 3, dans lequel le plan d'image de la seconde image est aligné avec la direction des faisceaux Doppler de la première image.

**5.** Système d'imagerie diagnostique ultrasonore selon la revendication 1, dans lequel la sonde à ultrasons est en outre conçue pour fonctionner en mode biplan pour balayer deux plans d'image de manière entrelacée dans le temps.

**6.** Système d'imagerie diagnostique ultrasonore selon la revendication 5, dans lequel la sonde à ultrasons est en outre conçue pour balayer deux plans d'image à un angle non orthogonal sélectionné par rapport au plan du transducteur à réseau matriciel.

7. Système d'imagerie diagnostique ultrasonore selon la revendication 5, dans lequel la sonde à ultrasons est en outre conçue pour balayer un plan d'image à un angle non orthogonal sélectionné par rapport à une direction d'écoulement.

8. Système d'imagerie diagnostique ultrasonore selon la revendication 1, dans lequel le calculateur de débit volumique est en outre conçu pour calculer un algorithme sous la forme

$$Q = \int_S v \cdot \mathrm{d}A$$

dans lequel Q est le débit volumique, v est la vitesse d'écoulement à angle corrigé, la surface S est un plan de coupe à travers un vaisseau contenant des données Doppler et A est l'aire de la lumière de vaisseau.

9. Système d'imagerie diagnostique ultrasonore selon la revendication 8, dans lequel le calculateur de débit volumique est en outre conçu pour additionner des valeurs de données Doppler à angle corrigé dans une lumière de vaisseau.

10. Procédé d'utilisation d'un système d'imagerie diagnostique ultrasonore pour effectuer un examen ultrasonore afin de mesurer le débit volumique comprenant :

le balayage (903) avec une sonde à ultrasons, la sonde comprenant un transducteur à réseau matriciel bidimensionnel, pour acquérir simultanément des données d'image Doppler à partir de deux plans d'image (90, 92) se croisant le long d'une direction de faisceau Doppler et produire à partir des données d'image Doppler deux images Doppler de débit, dans lequel la première image Doppler est celle d'un vaisseau cible dans une vue d'axe long et la seconde image Doppler est celle du vaisseau cible dans une vue transversale ;
l'affichage (907) simultané des deux images ;
en réponse à une commande utilisateur, l'affichage d'une ligne Doppler (82) et d'un curseur de débit (84) sur une première image (90) des images Doppler ;
la détermination de la correction d'angle (909) en fonction d'une direction de faisceau Doppler et d'une direction d'écoulement de la première image Doppler établie par la ligne Doppler et le curseur de débit ; et
le calcul du débit volumique (915) à partir des données de la seconde image Doppler en utilisant une correction d'angle déterminée à partir de la première image Doppler.

11. Procédé selon la revendication 10, dans lequel la sonde est conçue pour fonctionner en mode biplan pour acquérir la première image Doppler et la seconde image Doppler.

12. Procédé selon la revendication 10 ou 11, dans lequel la seconde image Doppler est dans un plan d'image aligné avec un angle Doppler de la première image Doppler.

13. Procédé selon la revendication 10, comprenant en outre l'ajustement de la direction de faisceau Doppler pour que la première image Doppler coupe la direction d'écoulement selon un angle non orthogonal, et

comprenant facultativement en outre la segmentation de l'écoulement dans la seconde image Doppler avec un modèle, et facultativement dans lequel le calcul du débit volumique comprend en outre l'intégration de pixels de valeur de débit du vaisseau cible dans la seconde image.

14. Système d'imagerie diagnostique ultrasonore pour analyser un débit volumique du sang comprenant :

un processeur de données d'image (24) sensible à une entrée provenant d'une sonde à ultrasons comprenant un transducteur à réseau matriciel bidimensionnel et conçu pour produire deux images Doppler de débit, ladite entrée comprenant des données d'image Doppler provenant de deux plans d'image (90, 92) se croisant le long d'une direction de faisceau Doppler acquise par la sonde à ultrasons simultanément, dans lequel une première des deux images présente une vue d'axe long d'un vaisseau, et la seconde des deux images présente une vue transversale du vaisseau ;
un écran (36) conçu pour afficher les deux images Doppler simultanément ;
un générateur graphique (49), sensible à une commande utilisateur, et conçu pour afficher une ligne Doppler (82) et un curseur de débit (84) sur une première image (90) des images Doppler ; et
un calculateur de débit volumique (40), sensible aux données d'image Doppler de la seconde image (92) des images Doppler et à un angle Doppler établi par la ligne Doppler et le curseur de débit, qui est conçu pour déterminer une mesure à angle corrigé du débit volumique.

FIG. 1

FIG. 2a

FIG. 2b

FIG. 3

FIG. 4

FIG. 5

FIG. 6

**FIG. 7**

**FIG. 7a**

FIG. 8

EP 4 142 605 B1

FIG. 8a

Start
901

Acquire 1st Doppler image
903

Adjust 1st image Doppler direction
904

Acquire 2nd Doppler image in Doppler direction of 1st image
905

Display both images
907

Determine Doppler angle correction for 1st image
909

Segment 2nd Doppler image
911

Calculate volume flow using 2nd image & angle corr. of 1st image
915

Display / record flow
920

FIG. 9

## REFERENCES CITED IN THE DESCRIPTION

### Patent documents cited in the description

- US 5769079 A
- EP 0842638 A
- US 2015250453 A
- US 6780155 B, Li
- US 6443896 B, Detmer
- US 6709394 B, Frisa
- US 7645237 B, Frisa
- US 5997479 A, Savord
- US 6013032 A, Savord
- US 6623432 B, Powers
- US 5833613 A, Averkiou

### Non-patent literature cited in the description

- **R. W. GILL**. Measurement of blood flow by ultrasound: accuracy and sources of error. *Ultrasound in Medicine and Biology*, 1985, vol. 11 (4), 625-641
- **O.D. KRIPFGANS et al.** Measurement of volumetric flow. *J. Ultrasound Med.*, 2006, vol. 25, 1305-1311
- **PICOT et al.** Rapid volume flow rate estimation using transverse colour Doppler imaging. *Ultrasound in Medicine and Biology*, 1995, vol. 21 (9), 1199-1209